Europäisches Patentamt

European Patent Office

Office européen des brevets

(19)

(11) Veröffentlichungsnummer: **0 233 455**
**B1**

# (12) EUROPÄISCHE PATENTSCHRIFT

(45) Veröffentlichungstag der Patentschrift:
09.05.90

(21) Anmeldenummer: **87100167.3**

(22) Anmeldetag: **08.01.87**

(51) Int. Cl.⁵: **C07B 31/00**
// C07C15/06, C07C47/00,
C07D333/06, C07C1/207,
C07C5/50, C07C209/00,
C07C255/00, C07C13/00,
C07C9/02, C07C211/00,
C07D335/02

(54) Hydrierung und Reduktion mit dem System Kohlenwasserstoff/Kohlenstoff.

(30) Priorität: **11.01.86 DE 3600609**

(43) Veröffentlichungstag der Anmeldung:
**26.08.87 Patentblatt 87/35**

(45) Bekanntmachung des Hinweises auf die Patenterteilung:
**09.05.90 Patentblatt 90/19**

(84) Benannte Vertragsstaaten:
**AT BE CH FR GB IT LI NL**

(56) Entgegenhaltungen:
**US-A- 2 626 286**

**SYNTHESIS, Nr. 1, Januar 1978, Seiten 23,24, Georg Thieme Publishers; L. BIN DIN et al.: "A simple method for reducing aromatic nitro-compounds, azo-compounds, and related systems"**

(73) Patentinhaber: **BASF Aktiengesellschaft,
Carl-Bosch-Strasse 38, D-6700 Ludwigshafen(DE)**

(72) Erfinder: **Dockner, Toni, Dr., Grossgasse 6,
D-6701 Meckenheim(DE)**
Erfinder: **Krug, Herbert, Mussbacher Strasse 49,
D-6700 Ludwigshafen(DE)**
Erfinder: **Sauerwald, Manfred, Dr., Gebhardtstrasse 16,
D-6701 Roedersheim-Gronau(DE)**

ACTORUM AG

## Beschreibung

Die Erfindung betrifft ein Verfahren zur Reduktion von Carbonylverbindungen oder von heteroaromatischen, kondensierten aromatischen oder kondensierten heteroaromatischen Verbindungen.

Von den vielen Reaktionen, die zur Reduktion organischer Verbindungen zur Verfügung stehen, bietet die katalytische Hydrierung den Vorteil großer Anwendungsbreite und experimenteller Einfachheit. Anstelle von molekularem Wasserstoff können auch dehydrierbare organische Verbindungen mit niedrigem Redoxpotential verwendet werden. Wasserstoffdonatoren für diese sogenannten katalytischen Transferhydrierungen sind insbesondere cyclische Olefine bzw. partiell hydrierte aromatische Kohlenwasserstoffe, sekundäre Alkohole oder Hydrazin. Als Lösungsmittel werden z.B. Kohlenwasserstoffe empfohlen, um Disproportionierungen der Wasserstoffüberträger zu vermeiden (Houben-Weyl, Methoden d. org. Chemie, 4. Aufl., Bd. IV/1c, S. 67 bis 76, 1980). Nachteilig an dem beschriebenen Verfahren ist die Notwendigkeit von Katalysatoren wie Raney-Nickel und insbesondere Edelmetallkatalysatoren, wobei Palladium bei Transferhydrierungen allen anderen Übergangsmetallen überlegen ist (s. L. M. Jackman, Advances in org. Chem. 2, 349–352, 1960).

Aus der US-A 2 626 286 ist ein Verfahren zur gleichzeitigen Dehydrierung bestimmter Carbocyclen zu Aromaten und Hydrierung von Olefinen zu Alkanen bei 750 bis 1100°F in Gegenwart von Aktivkohle bekannt.

In Synthesis, 1978, Seite 23 und 24 ist ein Verfahren zur Reduktion aromatischer Nitro- und Azoverbindungen beschrieben, wobei flüssige Paraffine, Leichtbenzin oder Cyclohexan als Wasserstoffdonatoren fungieren. Diese Reduktion zu Anilin bzw. Anilinderivaten erfolgt bei Temperaturen von 360–400°C mit Umsätzen von 66 bis 100% und Ausbeuten im Bereich von 28 bis 93%, bezogen auf umgesetzten Ausgangsstoff. Unter diesen Reaktionsbedingungen werden Ketone, Ester und Nitrilgruppen nicht reduziert. Schlechtere Ergebnisse wurden erzielt, wenn eine kontinuierliche Fahrweise gewählt und die Umsetzung bei Temperaturen von 380 bis 570°C in einer gepackten Kolonne durchgeführt wurde. Als Füllmaterial für die Kolonne wurden neben Glashelices, Aluminiumoxid und Siliciumoxid auch Tierkohle verwendet. Dabei lag der Umsatz für die Nitrobenzolreduktion im ersten Durchlauf bei 44% mit einer Ausbeute von nur 2% an Anilin. Bei erneuter Benutzung der Säule konnte die Ausbeute auf 50% gesteigert werden. Nachteilig an dem beschriebenen Verfahren ist die geringe Anwendungsbreite, da sich nur aromatische Verbindungen umsetzen lassen.

Der Erfindung lag nun die Aufgabe zugrunde, ein Reduktionsverfahren zu finden, das breit anwendbar ist, das aber die Nachteile katalytischer Verfahren, z.B. die Verwendung von Schwermetall- oder Edelmetallkatalysatoren, überwindet, das ferner billig ist, sich im großtechnischen Maßstab leicht durchführen läßt und möglichst hohe Umsätze bei hoher Selektivität liefert.

Demgemäß wurde ein Verfahren zur Reduktion von Carbonylverbindungen oder von heteroaromatischen, kondensierten aromatischen oder kondensierten heteroaromatischen Verbindungen gefunden, das sich dadurch auszeichnet, daß man diese Verbindungen mit Kohlenwasserstoffen in Gegenwart von Kohlenstoff bei Temperaturen von 150 bis 500°C umsetzt.

Nach dem erfindungsgemäßen Verfahren lassen sich Carbonylverbindungen oder heteroaromatische, kondensierte aromatische oder kondensierte heteroaromatische Verbindungen umsetzen. Die Anwendungsbreite des erfindungsgemäßen Verfahrens ist ausgesprochen groß. So können Aldehyde bzw. Ketone oder deren Derivate wie Ketale oder Thioketale zu Kohlenwasserstoffen und kondensierte aromatische Kohlenwasserstoffe zu Dehydroverbindungen reduziert werden.

Zur Reduktion von Carbonsäuren oder Estern sowie Nitrilen ist das Reduktionssystem wenig geeignet.

Carbonylfunktionen in Aldehyden oder Ketonen lassen sich hydrieren. So können beispielsweise Benzaldehyd zu Toluol, Cyclohexanon zu Cyclohexan, Benzophenon zu Diphenylmethan oder Acetessigester zu Ethylbutyrat reduziert werden.

Kondensierte aromatische oder heteroaromatische Verbindungen können unter Erhalt mindestens eines aromatischen Ringes hydriert werden. So entsteht z.B. aus Naphthalin 1,2,3,4-Tetrahydronaphthalin, aus Chinolin 1,2,3,4-Tetrahydrochinolin und aus Anthracen 1,2,3,4-Tetrahydroanthracen. Bei Verbindungen wie Benzimidazol, Benzothiazol oder Benzofuran wird die Doppelbindung im Heteroatom enthaltenden Ring reduziert.

Heteroaromatische Verbindungen wie Thiophen, Pyrrol, Furan oder Pyridin können vollständig hydriert werden zu den entsprechenden Tetrahydroverbindungen bzw. Piperidin.

Der für die Reduktion benötigte Wasserstoff entstammt dem Kohlenwasserstoff, aus dem neben wasserstoffärmeren Derivaten häufig Kohlenstoff als Endprodukt gebildet wird.

Als Kohlenwasserstoffe werden zweckmäßig hochsiedende Mineralöle verwendet, deren Siedetemperaturen höher liegen als die Reaktionstemperatur, die 150 bis 500, vorzugsweise 200 bis 400, insbesondere 250 bis 350°C beträgt. Solche Kohlenwasserstoffe sind z.B. Vakuumgasöl, technisches Weißöl, Heizöl S, Vakuumrückstandsöl oder sonstige bei der Erdölfraktionierung anfallende hochsiedende Bestandteile. Es ist auch möglich, niedrigersiedende Kohlenwasserstoffe wie Heizöl L, Benzin, Leichtbenzin, Cyclohexan oder niedere Kohlenwasserstoffe wie Methan, Ethan, Propan oder Butan zu verwenden (s. dazu Ullmann, Enzyklopädie d. techn. Chemie, 3. Auf., Bd. 6, S. 595 bis 760, 1955 und 4. Aufl., Bd. 12, S 570 bis 573). Allerdings ist es dann, um in der Flüssigphase arbeiten zu können, nötig, die Reaktion unter Druck durchzuführen. Rohöle verschiedener Herkunft sind ebenso einsetzbar.

Die Kohlenwasserstoffe werden in der Regel in einer Menge von 10 bis 2000 g, insbesondere 50 bis 1000 g pro Mol Ausgangsstoff verwendet.

Die Reaktion wird vorteilhaft in flüssiger Phase durchgeführt, wobei mindestens der Hauptteil der reagierenden Kohlenwasserstoffe flüssig vorliegen sollte. Die flüssige Phase enthält suspendierten Kohlenstoff, der entweder während der Reaktion entsteht, zugesetzt wird, oder als Bestandteil der Kohlenwasserstoffe, z.B. in Heizöl S oder Vakuumrückstand, vorhanden ist. Durch Zugabe von elementarem Kohlenstoff zum Reaktionsgemisch wird die Reaktion beschleunigt und der Umsatz gesteigert, insbesondere wenn der Kohlenwasserstoff keine oder nur geringe Mengen an Kohlenstoff enthält. Vorzugsweise liegen im Reaktionsgemisch 1 bis 50, insbesondere 5 bis 20 Gew.-% Kohlenstoff, bezogen auf den Kohlenwasserstoff vor. Geeignete Kohlenstoffzusätze sind z.B. Petrolkoks und Ruß oder eine andere Form des Graphits sowie Aktivkohlen wie Carboraffin P® oder Tierkohle, die z.B. mit $ZnCl_2$, Phosphorsäure oder Wasserstoff aktiviert sind.

Die Umsetzung kann vorteilhaft so ausgeführt werden, daß in eine Suspension von Kohlenstoff in Kohlenwasserstoff, der auf Reaktionstemperatur erhitzt ist, der zu reduzierende Stoff fest, flüssig oder gasförmig, gegebenenfalls zusammen mit einem Inertgas, z.B. Stickstoff, eingeführt wird. Bildet sich bei der Reaktion Wasser oder andere flüchtige Verbindungen, so können diese ggf. mit einem schwachen Inertgasstrom ausgetrieben werden. Je nach Siedepunkt verläßt das Produkt gasförmig das Reaktionsgefäß bereits während der Durchführung der Reaktion; es kann dann kondensiert und nach bekannten Methoden, z.B. destillativ, aufgearbeitet werden. Produkte mit höherem Siedepunkt verbleiben im Öl und werden nach der Umsetzung z.B. durch Destillation oder Extraktion nach dafür üblichen Techniken isoliert.

Die erfindungsgemäße Umsetzung kann diskontinuierlich oder kontinuierlich bei Normaldruck, erhöhtem oder vermindertem Druck nach dafür gebräuchlichen Methoden z.B. in einem Rührreaktor oder in einem zylindrischen Reaktor mit Umlauf durchgeführt werden.

### Beispiel 1

Umsetzung von Benzaldehyd zu Toluol

Wie in Beispiel 1 beschrieben, wurden 225 g technisches Weißöl und 25 g Aktivkohlepulver auf 350° erhitzt und stündlich 10,5 g Benzaldehyd mit ca. 5 l Stickstoff zudosiert.

Nach 3 Stunden erhielt man 33,7 g Kondensat, das laut gaschromatographischer Analyse des Destillates 18,6% Toluol und 69,3% Benzaldehyd enthielt. Der Umsatz betrug demnach 25,8%, die Selektivität 88,6%.

Bei gleicher Versuchsdurchführung, jedoch ohne Aktivkohlezusatz erfolgte keine Umsetzung.

### Beispiel 2

Umsetzung von Cyclohexanon zu Cyclohexan

Wie in Beispiel 1 beschrieben, wurden 450 g technisches Weißöl und 50 g Aktivkohle auf 350°C erhitzt und stündlich 30 g Cyclohexanon mit 10 l Stickstoff zudosiert.

Nach 2 Stunden erhielt man 62 g Kondensat, das laut gaschromatographischer Analyse 55 g Cyclohexanon enthielt. Der Umsatz betrug demnach 8,3%, die Selektivität 93,3%.

### Beispiel 3

Umsetzung von Naphthalin zu 1,2,3,4-Tetrahydronaphthalin

In einem Druckreaktor wurden 800 g technisches Weißöl, 200 g Carboraffin P® und 125 g Naphthalin 6 Stunden bei 360°C gerührt. Nach Abkühlen wurde der Autoklaveninhalt in eine Destillationsapparatur überführt und bis zu einer Sumpftemperatur von 340°C abdestilliert.

Man erhielt 179 g Destillat mit 49 Gew.-% 1,2,3,4-Tetrahydronaphthalin, entsrechend einem Umsatz von 70,2%. Die Ausbeute an 1,2,3,4-Tetrahydronaphthalin betrug 90%, bezogen auf umgesetztes Naphthalin.

### Beispiel 4

Umsetzung von Thiophen zu Tetrahydrothiophen

In einem Druckreaktor wurden 400 g technisches Weißöl, 50 g Aktivkohle und 84 g Thiophen gemischt und unter Rühren 2 Stunden auf 340 bis 355°C erhitzt. Nach Abkühlen auf Raumtemperatur wurde der Reaktorinhalt in eine Destillationsapparatur überführt und bis zu einer Sumpftemperatur von 200°C abdestilliert.

Man erhielt 79 g Destillat, das laut gaschromatographischer Analyse 29 g Thiophen und 36 g Tetrahydrothiophen enthielt.

Der Umsatz betrug demnach 65,5%, die Selektivität 62,5%.

### Patentansprüche

1. Verfahren zur Reduktion von Carbonylverbindungen oder von heteroaromatischen, kondensierten aromatischen oder kondensierten heteroaromatischen Verbindungen, dadurch gekennzeichnet, daß man diese Verbindungen mit Kohlenwasserstoffen in Gegenwart von Kohlenstoff bei Temperaturen von 150 bis 500°C umsetzt.

2. Verfahren gemäß Anspruch 1, dadurch gekennzeichnet, daß man als Kohlenwasserstoff hochsiedende Mineralöle verwendet, deren Siedepunkte höher liegen als die Reaktionstemperatur.

3. Verfahren gemäß den Ansprüchen 1 und 2, dadurch gekennzeichnet, daß man als Kohlenwasserstoff Vakuumrückstand, Heizöl S oder technisches Weißöl verwendet.

4. Verfahren gemäß Anspruch 1, dadurch gekennzeichnet, daß man niedrigsiedende Kohlenwasserstoffe wie Heizöl L, Benzin, Leichtbenzin oder niedere Kohlenwasserstoffe wie Methan, Ethan, Propan oder Butan verwendet und die Umsetzung unter erhöhtem Druck durchführt.

5. Verfahren gemäß den Ansprüchen 1 bis 4, dadurch gekennzeichnet, daß man den Kohlenstoff in einer Menge von 1 bis 50 Gew.-%, bezogen auf den Kohlenwasserstoff, verwendet.

6. Verfahren gemäß den Ansprüchen 1 bis 5, dadurch gekennzeichnet, daß man kondensierte aromatische oder heteroaromatische Verbindungen umsetzt.

7. Verfahren gemäß den Ansprüchen 1 bis 5, dadurch gekennzeichnet, daß man heteroaromatische Verbindungen umsetzt.

8. Verfahren gemäß den Ansprüchen 1 bis 5, dadurch gekennzeichnet, daß man Carbonylverbindungen umsetzt.

## Claims

1. A process for reducing carbonyl compounds or heteroaromatic, fused aromatic or fused heteroaromatic compounds, which comprises reacting these compounds with hydrocarbons in the presence of carbon at 150–500°C.

2. A process as claimed in claim 1, wherein the hydrocarbon used is a high-boiling mineral oil with a boiling point higher than the reaction temperature.

3. A process as claimed in claims 1 and 2, wherein the hydrocarbon used is vacuum residue, heavy fuel oil or technical-grade white oil.

4. A process as claimed in claim 1, wherein low-boiling hydrocarbons such as light fuel oil, gasoline, light naphtha or lower hydrocarbons such as methane, ethane, propane or butane are used and the reaction is carried out under superatmospheric pressure.

5. A process as claimed in any of claims 1 to 4, wherein the carbon is used in an amount of from 1 to 50% by weight, based on the hydrocarbon.

6. A process as claimed in any of claims 1 to 5, wherein fused aromatic or heteroaromatic compounds are reacted.

7. A process as claimed in any of claims 1 to 5, wherein heteroaromatic compounds are reacted.

8. A process as claimed in any of claims 1 to 5, wherein carbonyl compounds are reacted.

## Revendications

1. Procédé de réduction de composé carbonylés ou de composés hétéro-aromatiques, aromatiques condensés ou hétéro-aromatiques condensés, caractérisé en ce qu'on fait réagir ces composés avec des hydrocarbures en présence de carbone à des températures de 150 à 500°C.

2. Procédé selon la revendication 1, caractérisé en ce qu'on utilise, comme hydrocarbure, des huiles minérales de point d'ébullition élevé, dont les points d'ébullition se situent plus haut que la température de la réaction.

3. Procédé selon la revendication 1 ou 2, caractérisé en ce qu'on utilise, comme hydrocarbure, un résidu court de la distillation poussée, une huile lourde ou une huile blanche technique.

4. Procédé selon la revendication 1, caractérisé en ce qu'on utilise des hydrocarbures de bas point d'ébullition tels que le fioul léger, l'essence ordinaire, le white-spirit ou des hydrocarbures inférieurs tels que le méthane, l'éthane, le propane ou le butane et on conduit la réaction sous pression élevée.

5. Procédé selon l'une quelconque des revendications 1 à 4, caractérisé en ce qu'on utilise le carbone dans une proportion de 1 à 50% en poids par rapport à l'hydrocarbure.

6. Procédé selon l'une quelconque des revendications 1 à 5, caractérisé en ce qu'on met en réaction des composés aromatiques ou hétéro-aromatiques condensés.

7. Procédé selon l'une quelconque des revendications 1 à 5, caractérisé en ce qu'on met en réaction des composés hétéro-aromatiques.

8. Procédé selon l'une quelconque des revendications 1 à 5, caractérisé en ce qu'on met en réaction des composés carbonylés.